# EUROPEAN PATENT SPECIFICATION

(11) **EP 1 352 030 B1**
(45) Date of publication and mention of the grant of the patent: **21.12.2005**
(21) Application number: 01910739.0
(22) Date of filing: 15.02.2001
(51) Int. Cl.: C08L 77/00, C08L 77/06

(54) **IMPACT MODIFIED POLYAMIDE COMPOSITION**
SCHLAGZÄHMODIFIZIERTE POLYAMID-ZUSAMMENSETZUNG
COMPOSITION POLYAMIDE ANTICHOC

(30) Priority: 19.01.2001 US 765749
(43) Date of publication of application: 15.10.2003
(73) Proprietor: E.I. DUPONT DE NEMOURS AND COMPANY, Wilmington, DE 19898 (US)
(72) Inventor: DIBENEDETTO, Silvia, CH-1196 Gland, (CH); FLEXMAN, Edmund, A., Wilmington, DE 19810 (US)
(74) Representative: Freiherr von Wittgenstein, Arved
(86) International application number: PCT/US2001/004861
(87) International publication number: WO 2002/057366

(56) References cited:
- WO-A-01/53415
- WO-A-98/38227
- GB-A- 1 552 352
- US-A- 4 485 214

## Description

### BACKGROUND OF THE INVENTION

### Field of the Invention

This invention relates to the field of toughened polyamides, and more particularly to the field of polyamides that are toughened by the addition of tougheners such as ionomers and ethylene copolymers.

### Description of the Related Art

Improvement of impact strength, or toughness, of polyamide resins has long been of interest. Resistance to shattering or brittle breaking on impact of polyamide molded articles is a desirable feature of any molded article. Any tendency to break on impact in a brittle fashion (rather than ductile fashion) is a significant limitation on the usefulness of such articles. By "ductile" is meant that sharp cracks in the molded resin are not initiated, or if initiated do not tend to propagate, from the area of impact. Breaks in ductile materials are characterized more by tearing with a large volume of adjacent material yielding at the edge of the crack or tearing rather than a sharp, clean break with little molecular displacement. A resin having good ductility is one that is resistant to crack propagation caused by impact.

A variety of additives have been added to polyamide resins to improve strength and ductility. For example, U.S. Pat. No. 4,174,358 issued Nov. 13, 1979 to Epstein, describes improving impact strength and ductility by adding a selected random copolymer which adheres to the polyamide. U.S. Pat. No. 4,594,386 issued Jun. 10, 1986 to Olivier, describes improving impact strength and toughness of polyamide resins by blending polyamide resin with maleic anhydride grafted EPM rubber of low molecular weight. U.S. Pat. No. 4,346,194 issued Aug. 24, 1982 to Roura, describes a polyamide molding material having good impact strength at low temperatures, e.g., 0° C.; the polyamide molding material comprises a nylon 6/66 blend and a toughening copolymer that is an adduct of a polymer of ethylene, at least one C3-C6 alpha-olefin and at least one non-conjugated diene, with an unsaturated compound containing carboxyl or carboxyl derivatives.

The inverse relationship between percent neutralization and ionic polymer flow rate, such as might be measured by melt index, i.e., M.I., is well known. This inverse relationship can present a processing problem in cases where it may be desired to process a highly neutralized ionomer resin that contains even moderate levels of methacrylic acid. While it can be inferred that a higher degree of neutralization of acid groups in the resin can lead to greater toughening, ultimate flow rates at the highest levels of neutralization, as measured by M.I., approach a "no flow" condition, i.e., the resin blend becomes intractable. In US Patent No. 5,688,868 issued November 18, 1997 to Fish, there is disclosed a process for preparing blends of polyamide and ionic copolymers which have very high degrees of neutralization by adding metal ions to the blend in situ in amount which exceed that needed to neutralize 100% of the acid molecules present in the blend without incurring a "no flow" condition.

In addition, US Patent Nos. 4,346,194 and 4,478,978 to Roura disclose toughened blends of nylon 6,6 and nylon 6.

Although the previously known combinations impart improved toughness to polyamide resins, it has now been found that certain novel compositions provide further improvements in polyamide resin toughness at very low temperatures, including temperatures less than about -30° C. This invention is directed to a polyamide molding blend suitable for making molded articles for use in applications requiring toughness at very low temperatures.

The present invention is directed to the discovery that the toughness of a polyamide that includes an EP or EPDM grafted with 0.05 to 3 weight percent of a carboxylic acid or an anhydride thereof can be unexpectedly increased by the addition of a particular ionomer at least partially dispersed in nylon 6, provided that the number of moles of selected metal ions in the ionomer is greater than or equal to the number of moles of the carboxylic acid in the grafted EP or EPDM.

### SUMMARY OF THE INVENTION

The present invention relates to a multiphase polymer composition having increased impact resistance at low temperatures, that includes a blend of
(1) an ionomer at least partially dispersed in nylon 6, the ionomer being formed from a partially neutralized ethylene acid interpolymer precursor, the ethylene acid interpolymer precursor having polymerized in-chain units derived from the monomers comprising:
   (a) ethylene,
   (b) 2 to 25 weight percent of an acid selected from the group of acrylic acid, methacrylic acid, and mixtures thereof,
   (c) 0.1 to 15 weight percent of a dicarboxylic acid monomer selected from the group consisting of maleic acid, fumaric acid, itaconic acid, maleic anhydride, itaconic anhydride, a C₁-C₄-alkyl half ester of maleic acid, and a mixture of these dicarboxylic acid monomers,
   (d) 0 - 40 weight percent of a C1-C8-alkyl alkyl acrylate,
   the ionomer being formed by neutralization of from about 5 to 90 percent of the total number of carboxylic acid units in the copolymer with metal ions selected from the group of zinc, magnesium, manganese and mixtures thereof, alone or in combination with sodium or lithium ions,
   with the proviso that the total of acrylic acid, methacrylic acid, and dicarboxylic acid monomer is from 4 to 26 weight percent of the acid copolymer precursor,
   the ionomer having a melt index of from 0.01 to 100 grams/10 minutes; and
(2) a polyamide other than nylon 6, and
(3) a composition that includes an elastomer selected from the group of EP, EPDM and styrenic thermoplastic elastomer, which elastomer is grafted with 0.05 to 3 weight percent of a carboxylic acid or any anhydride thereof;
   wherein the ratio of the number of moles of metal ions in the ionomer to the number of moles of the carboxylic acid or any anhydride thereof in the grafted EP, EPDM or styrenic thermoplastic elastomer is greater than or equal to 1.0.

### BRIEF DESCRIPTION OF THE DRAWINGS

The figures are TEM images produced by cutting sections of molded bars or pellets perpendicular to the melt flow direction using a diamond knife. The sections were accumulated in cold ethanol, transferred to 1% phosphotungstic acid for overnight staining, rinsed, and captured on copper mesh grids. The images were recorded using a JEOP 1200 EX TEM operated at 100 KV accelerating voltage and recorded on sheet film. The scale of the image is evident from the micron bar shown on the image.

Figure 1 is a TEM image of a composition of nylon 6 and a neutralized ionomer of ethylene, methacrylic acid and maleic acid monethyl ester.

Figure 2 is a TEM image of a composition nylon 6, a neutralized ionomer of ethylene, methacrylic acid and maleic acid monethyl ester, Nylon 6,6, and EP grafted with maleic anhydride.

Figure 3 is a TEM image of Zytel® ST801 NC01A impact modified nylon 6,6.

Figure 4 is a TEM image of a composition a neutralized ionomer of ethylene, methacrylic acid and maleic acid monethyl ester, nylon 6,6, and EP grafted with maleic anhydride.

Figure 5 is a TEM image of a composition nylon 6, a neutralized ionomer of ethylene, methacrylic acid and maleic acid monethyl ester, and EP grafted with maleic anhydride.

### DEFINITIONS

The term "polymer" as used herein, generally includes but is not limited to, homopolymers, copolymers (such as for example, block, graft, random and alternating copolymers), terpolymers, etc. and blends and modifications thereof. Furthermore, unless otherwise specifically limited, the term "polymer" shall include all possible geometrical configurations of the material. These configurations include, but are not limited to isotactic, syndiotactic and random symmetries.

The term "EPDM" refers to ethylene propylene diene monomer elastomers and is used herein to mean any elastomer that is a terpolymer of ethylene, an alpha-olefin having from 3 to 10 carbon atoms, and a copolymerizable non-conjugated diene such as 5-ethylidene-2-norbomene, dicyclopentadiene, 1,4-hexadiene, and the like.

The term "EP" as used herein means any copolymer or terpolymer of ethylene and an alpha-olefin having from 3 to 10 carbon atoms, such as EPR, EPM, or an ethylene propylene copolymer.

### DETAILED DESCRIPTION

The present invention relates to a multiphase polymer composition having increased impact resistance at low temperatures.

The inventive composition is a multiphase composition that includes a blend of (1) an ionomer at least partially dispersed in nylon 6, (2) a polyamide other than nylon 6, and (3) an EP or EPDM grafted with a carboxylic acid or any anhydride thereof, wherein the ratio of the number of moles of metal ions in the ionomer to the number of moles of the carboxylic acid or any anhydride thereof in the grafted EP or EPDM is greater than or equal to 1.0. The composition preferably includes from 10 to 50 weight percent nylon 6, from 1 to 30 weight percent of an ionomer of ethylene, methacrylic acid and maleic acid monoethyl ester, from 35 to 90 weight percent of a nylon other than nylon 6,6, and from 5 to 25 weight percent of EP or EPDM grafted with carboxylic acid or any anhydride thereof, based on the total weight of the composition.

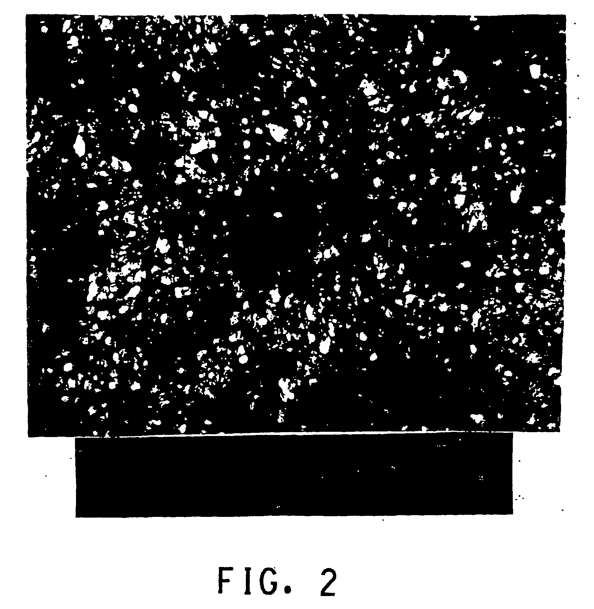

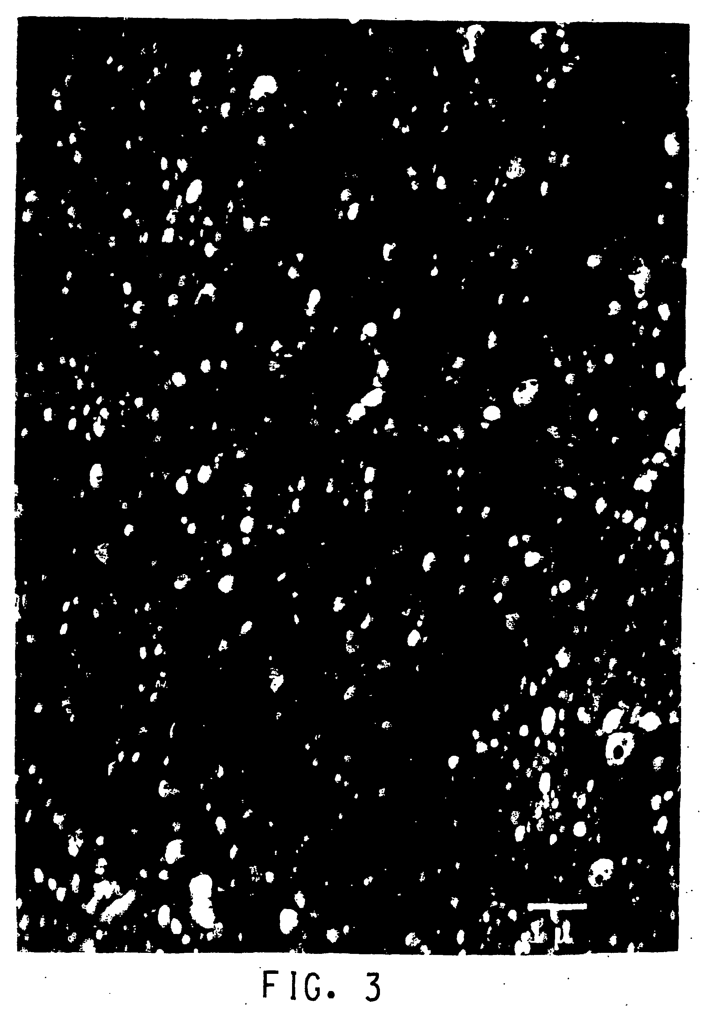

Without wishing to be bound by theory, it is believed that the composition of the invention enables a morphology that, when used in molded polyamide articles, is believed to result polyamide in articles that exhibit a significant increase in the ductility at low temperatures. With the composition of the invention, both a network of nylon in the elastomer and a network of elastomer in nylon is formed. As can be seen in Figure 2, the morphology of an article made with a composition according to one preferred embodiment of the invention resembles interpenetrating polymer networks. The morphology of a molded article made with a conventional elastomer-toughened polyamide composition, as shown in Figure 3, shows approximately spherical elastomer particles in a nylon matrix.

The ionomer of the composition of the invention is formed from a partially neutralized acid copolymer precursor (or the acid copolymer can be neutralized during dispersion), the acid copolymer precursor having polymerized in-chain units derived from the monomers comprising:
(a) ethylene,
(b) 2 to 25 weight percent of an acid selected from the group of acrylic acid, methacrylic acid and mixtures thereof,
(c) 0.1 to 15 weight percent of a dicarboxylic acid monomer selected from the group consisting of maleic acid, fumaric acid, itaconic acid, maleic anhydride, itaconic anhydride, a C₁-C₄-alkyl half ester of maleic acid, and a mixture of these dicarboxylic acid monomers,
(d) 0 - 40 weight percent of a C1-C8-alkyl alkyl acrylate,
   the ionomer being formed by neutralization of from about 5 to 90 percent of the total number of carboxylic acid units in the copolymer with metal ions selected from the group of zinc, magnesium, manganese and mixtures thereof, alone or in combination with sodium or lithium ions (this neutralization level is the level prior to the compounding with the nylon 6).

Preferably, the total of (meth)acrylic acid and dicarboxylic acid monomer is from 4 to 26 weight percent of the acid copolymer precursor. It is further preferred that the total co-monomer content not exceed 50 weight percent of the acid copolymer precursor. The preferred ionomer has a melt index of from 0.01 to 100 grams/10 minutes.

This ionomer and its preparation are generally disclosed in International Application Number PCT/US98/03611, International Publication Number WO 98/38227, filed in the name of E.I. du Pont de Nemours and Company, the text of which is hereby incorporated by reference.

In referring to percent neutralization of acid units of the ionomer, a monocarboxylic acid provides one acid unit, a dicarboxylic acid provides two acid units, an anhydride such as maleic anhydride is considered to provide two acid units, and half esters are considered to provide one acid unit. The calculation of percent neutralization is based on the number of acid units considered to be present as per above, and the number of metal equivalents added. In fact, anhydride units may remain as anhydride units rather than be changed to acid units. When subject to neutralization, an anhydride monomer unit may form a di-metal salt, a mono-metal salt, form an un-neutralized diacid unit, or leave the anhydride unit unaltered as an anhydride unit, acting as if it had no acid functionality. The half esters of diacids, while counted as having only one acid, may actually be converted to diacids or anhydrides, with the various possibilities related to neutralization noted above. The presence of water under pressure in the melt is one factor affecting these possibilities. As stated however, whatever the number of acid groups (free or neutralized) actually present, the calculated percent neutralization is based on the number of acid units considered to be provided as defined above. In view of the various 'mutations' of the diacid monomers and salts possible, the actual percent of neutralized acid groups as a percent of actual total neutralized and non-neutralized free acid groups may therefore differ from the calculated percent neutralization, which is based on the above assumptions. The difference is due to anhydride units which are not acid units, but are counted as two acid units.

The total percent neutralization, as defined in the above paragraphs, is from about 5 to 90 percent, preferably 10 to 70 percent, most preferably between 25 and 60 percent. While lower neutralization levels will provide less ionomer character, higher levels will produce lower flow ionomers.

The polyamide other than nylon 6 that is used in the composition of the invention may be nylon-6,6, nylon-6,10, nylon-6/66, nylon-6,12, nylon - 12, nylon-11 or any high temperature nylon (polyphtalamide).

The grafted EP or EPDM is an EP or EPDM grafted with 0.05 to 3 weight percent of a carboxylic acid or any potential anhydride thereof. Preferably, the carboxylic acid or anhydride thereof is selected from the group consisting of maleic acid, fumaric acid, itaconic acid, maleic anhydride, itaconic anhydride, a C₁-C₄-alkyl half ester of maleic acid, and mixtures thereof. It is recognized that anhydride containing styrenic thermoplastic elastomers are functionally equivalent to the above grafted EP or EPDM elastomers.

The metal ions in the ionomer are selected from the group of zinc, magnesium, manganese and mixtures thereof, alone or in combination with sodium or lithium ions. A preferred metal ion is zinc.

The ratio of the number of moles of metal ions in the ionomer to the number of moles of the carboxylic acid or an anhydride thereof in the grafted EP or EPDM is greater than or equal to 1.0, and preferably greater than 1.5. There is no theoretical upper limit for this ratio, although a practical upper limit is 3.0. In addition to a kinetic limitation with regard to the percent of actual neutralization as opposed to the theoretically possible due to limited time in the mixing device, the reason for a practical upper limit is as follows. The ratio of the number of moles of metal ions in the ionomer to the number of moles of the carboxylic acid in the grafted EP or EPDM can be increased either by increasing the ionomer component or by decreasing the grafted EP or EPDM component. In the first case, when a large quantity of the ionomer in nylon 6 is used, it will leave only a small percentage of the nylon other than nylon 6 in which the grafted EP or EPDM has to dispersed. This would result in poor dispersion of the EP or EPDM. In the second case, where the grafted EP or EPDM is decreased, the reduced number of grafting sites will undesirably reduce the overall toughness of the material as it is known that low temperature impact resistance increases with concentration of grafting sites.

Without being bound to any particular theory, it is believed that the metal ions in the ionomer of the composition, and in particular the excess metal ions, interact with the some of the carboxylic acid or anhydride thereof that is grafted on the EP or EPDM. This interaction can be compared to the neutralization of the acid units of the ionomer by the metal ions as described above. In fact, the carboxylic acid or anhydride thereof grafted on the EP or EPDM can either react with the amine ends of the nylon or interact with the metal ions. It is believed that the interaction between the ionomer, the nylon 6, and the grafted EP or EPDM, when dispersed in the nylon other than nylon 6 generates a morphology which confers improved toughness and that looks like an interpenetrating network system.

The compositions of the present invention may be prepared by melt blending the ionomer and nylon 6, and then melt blending this composition with the polyamide other than nylon 6 and the EP or EPDM grafted with a carboxylic acid or anhydride thereof. The inventive composition may be made by admixing the components in the desired proportions and melt blending the admixture under high shear in conventional mixing equipment, such as an extruder, Banbury mill, Buss Kneader, Farrell Continuous Mixer or the like. The components are preferably combined with one another via simultaneous metering of the component streams. Pellet blends of the components can also be directly injection molded if sufficient mixing is present in the molding machine from back pressure, a mixing screw or head, static mixers in the die, or the like.

Blends prepared according to this invention may also contain one or more conventional additives, such as stabilizers and inhibitors of oxidative, thermal, and ultraviolet light degradation; lubricants and mold release agents; colorants including dyes and pigments; flame-retardants; fibrous and particulate fillers and reinforcements; plasticizers, processing aids, and the like. These additives are ordinarily added during the mixing step before melt blending or during melt blending.

The invention is further illustrated by the following examples. It will be appreciated that the examples are for illustrative purposes only and are not intended to limit the invention as described above. Modification of detail may be made without departing from the scope of the invention.

### EXAMPLE 1

The composition of Examples 1A and 1B were made by blending nylon-6, an ionomer, nylon 6,6, and EP grafted with maleic anhydride.

The composition of Example 1A was made by forming a melt blend of the nylon-6 with the ionomer in a twin screw extruder and subsequently blending this first melt blend with the nylon 6,6 and the EP grafted with maleic anhydride in a twin screw extruder to form the complete composition.

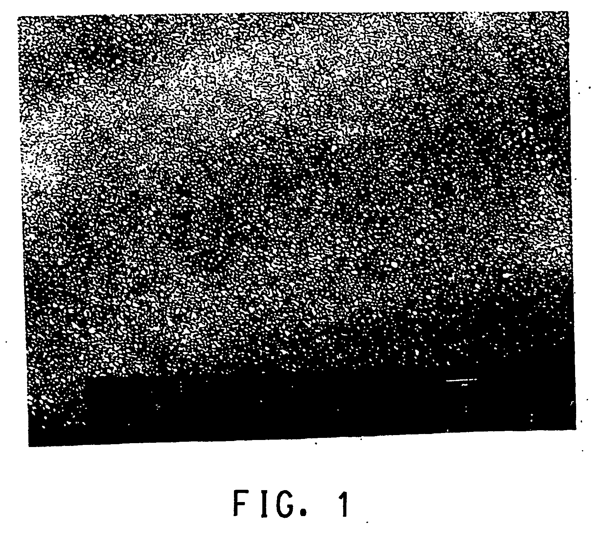

The ionomer was a terpolymer of about 83 weight percent ethylene, 11 weight percent methacrylic acid, and 6 weight percent maleic acid monethyl ester having 40% neutralisation by Zinc, that was prepared as generally disclosed in International Application Number PCT/US98/03611. This ionomer was blended with nylon-6 (Viscosity Number ("VN") 131-153 in sulfuric acid, according to DIN 53727), at a ratio of 30 weight percent ionomer to 70 weight percent nylon-6, in a Berstorff twin screw extruder having a diameter of 40mm and an L/D ratio of 33. The barrel temperature at set up was 270°C at the hopper end and 240°C towards the die end, and the die had a set-up temperature of 260°C. The screw was turned at 300 rpm and the two components were added to the extruder and combined via simultaneous addition to the extruder in a single pass. TEM images of this first blend (accumulated in cold ethanol and transferred to 1% phosphotungstic acid) showed a very evenly-sized dispersion of approximately spherical elastomer particles with an average particle size of 0.014 to 0.07 µm. A TEM image of this blend is shown in Figure 1.

The complete composition of Example 1A was formed by melt blending 26 weight percent of the first blend described above with 55 weight percent nylon 6,6 (VN 138 to 149 in Formic acid, according to ISO307) and with 19 weight percent EP grafted with maleic anhydride (MFI 20 @ 280°C, 2.16Kg; 0.9% MAh). This complete composition was blended according to the same process conditions used to produce the first blend except that the barrel temperature at set up was 300°C at the hopper end and 270°C towards the die end, and the die had a set-up temperature of 280°C. The ratio of the moles of zinc to moles of maleic anhydride in this complete composition was 1.5, which is calculated by taking the number of moles of zinc in the ionomer and dividing that by the number of moles of maleic anhydride grafted onto the EP. As used herein, the percent neutralization is equal to this ratio multiplied by 100.

In Example 1B, the nylon-6 (VN 131-153 in sulfuric acid, according to DIN 53727) and the ionomer of Example 1A were directly dry-blended with the nylon 6,6 and the EP grafted with maleic anhydride in a twin screw extruder at the following ratio to form the complete composition: 18.2 weight percent of nylon-6, 7.8 weight percent of the ionomer, 55 weight percent nylon 6,6, and 19 weight percent EP grafted with maleic anhydride. The complete composition of Example 1B was blended under the same process conditions that were used to produce the complete composition of Example 1A.

Test flex bars (5 to 10 bars per example per test) of the inventive composition, blended according to the processes of Examples I A and 1B, were molded according to ISO 294 in a standard injection molding machine with a melt temperature of about 285°C, a mold temperature of about 75°C, and a hold pressure of 85 MPa. The molded flex bars were notched according to specimen type 1A in ISO 180 and were tested according to the Notched Izod (NI) test method ISO 180 at 23°C in the dry-as-molded state (DAM) and in the 50% relative humidity conditioned state (50% RH). Flex bars in the DAM state were notched and tested according to the same test methods at -30°C and -40°C. The average impact energy measurements as per ISO 180 are reported in units of KJ/m² in Table 1.

**Table 1.**

| | Example 1A (Melt pre-blend) | Example 1B (Dry-blend) |
|---|---|---|
| Notched Izod 23°C DAM | 89 | 90 |
| Notched Izod 23°C 50% RH | 95 | 89 |
| Notched Izod -30°C DAM | 75 | 77.4 |
| Notched Izod -40°C DAM | 36.6 | 37.5 |

The test results in Table 1 show that the composition of this invention can be prepared by pre-blending the nylon-6 with the ionomer and subsequently blending this first blend with the nylon 6,6 and the EP grafted with maleic anhydride, or it can be prepared by directly dry-blending nylon-6 and the ionomer with the nylon 6,6 and the EP grafted with maleic anhydride without significantly changing the ductility of the material. TEM images of samples in Table 1 (accumulated in cold ethanol and transferred to 1% phosphotungstic acid) showed a morphology which looks like interpenetrating networks (IPN) with an average particle size of 0.04 to 0.3 m. A TEM image of the composition of Example 1A is shown in Figure 2.

### COMPARATIVE EXAMPLE 2

In Comparative Example 2, molded flex bars consisting 100% of the commercially available impact modified nylon 6,6 product Zytel® ST801 NC01A, available from E.I du Pont de Nemours and Company, Wilmington, Delaware, were tested for impact resistance. The test flex bars (5 to 10 bars per test) were molded according to ISO 294 in a standard injection molding machine with a melt temperature of about 285°C, a mold temperature of about 75°C, and a hold pressure was 85 MPa.

The molded flex bars as described above were notched according to specimen type 1A in ISO 180 and tested in the dry-as-molded state according to the Notched Izod test used in Example 1 at 23°C, -30°C and -40°C. The average impact energy measurements as per ISO 180 are reported in units of KJ/m² in Table 2.

**Table 2.**

| | Comp. Example 2 (ST801 NC101A) | EXAMPLE 1A (melt pre-blend) | Example 1B (dry-blend) |
|---|---|---|---|
| Notched Izod 23°C DAM | 85 | 89.0 | 90.0 |
| Notched Izod -30°C DAM | 23 | 75 | 77.4 |
| Notched Izod -40°C DAM | 19 | 36.6 | 37.5 |

The results in Table 2 show that at low temperatures, the composition of the invention (Example 1) provides a further improvement in ductility over a well known and commercially available impact modified polyamide composition, as represented by an increase in Notched Izod of more than 300% at -30°C compared to the compositions of the Zytel® ST801 NC01A at -30°C, dry as molded. Figure 3 shows a TEM image of Zytel® ST801 NC010A (accumulated in cold ethanol and transferred to 1% phosphotungstic acid) in which nearly spherical elastomer particles of average particle size between 0.04 and 0.9 µm dispersed in the nylon matrix can be seen.

### EXAMPLE 3

Compositions were made according to the process of Example 1A except that the relative weight percents of the the nylon-6, the ionomer, and the nylon 6,6 were varied as shown in Table 3 to change the level of neutralization. Molded flex bars comprised of the compositions 3A, 3B and 3C, were tested for impact resistance according to the Notched Izod test method used in Example 1. The test flex bars (5 to 10 bars per test) were molded according to ISO 294 in a standard injection molding machine with a melt temperature of about 285°C, a mold temperature of about 75°C, and a hold pressure was 85 MPa. The bars were tested in the dry-as-molded state at 23°C, -30°C and -40°C. The average impact energy measurements as per ISO 180 for Examples 3A, 3B and 3C, as well as for Example 1A are reported in units of KJ/m² in Table 3

**Table 3.**

| Example | 3A | 3B | 1A | 3C |
|---|---|---|---|---|
| Composition (weight %) | | | | |
| Nylon 6 | 6.1 | 12.3 | 18.2 | 24.5 |
| Ionomer | 2.6 | 5.2 | 7.8 | 10.5 |
| Nylon 6,6 | 72.3 | 63.5 | 55 | 46 |
| g-Mah-EP | 19 | 19 | 19 | 19 |

| % Neutralization | 50 | 100 | 150 | 200 |
|---|---|---|---|---|
| Properties | | | | |
| Notched Izod 23°C DAM | 85.2 | 82.3 | 89.0 | 92.9 |
| Notched Izod -30°C DAM | 24.6 | 67.5 | 75 | 82.1 |
| Notched Izod -40°C DAM | 17.6 | 31.7 | 36.6 | 41.5 |

The results in Table 3 show that a level of neutralization of about 100% is desired to achieve the desired improvement in ductility at -30°C. A preferred level of neutralization that achieves superior impact resistance at -30°C is 150%.

### EXAMPLE 4

Compositions were made according to the process of Example 1A except that the total elastomer modifier level was varied, at two neutralization levels, namely at 50% and 150%. The total elastomer modifier level was the combined weight percent of the ionomer of Example 1 and the maleic anhydride-grafted EP (MFI 20 @ 280°C, 2.16Kg; 0.9% MAh). The level of maleic anhydride was also varied within each level of neutralization. Molded flex bars comprised of the compositions 4A, 4B and 4C were tested for impact resistance according to the Notched Izod test method used in Example 1. The test flex bars (5 to 10 bars per test) were molded according to ISO 294 in a standard injection molding machine with a melt temperature of about 285°C, a mold temperature of about 75°C, and a hold pressure was 85 MPa. The bars were tested in the dry-as-molded state at 23°C, -30°C and -40°C. The average impact energy measurements as per ISO 180 are reported in units of KJ/m² in Table 4 and are compared against Examples 1A and 3C.

**Table 4.**

| Example | 4A | 4B | 4C | 1A | 3B |
|---|---|---|---|---|---|
| Composition (weight %) Nylon 6 | 3.1 | 6.1 | 9.1 | 18.2 | 12.3 |
| Ionomer | 1.3 | 2.6 | 3.9 | 7.8 | 5.2 |
| Nylon 6,6 | 86.1 | 72.3 | 77.5 | 55.0 | 63.5 |
| g-Mah-EP | 9.5 | 19.0 | 9.5 | 19.0 | 19.0 |
| Total elastomer % | 10.8 | 21.6 | 13.4 | 26.8 | 24.2 |
| % Neutralisation | 50 | 50 | 150 | 150 | 100 |

| Properties | | | | | |
|---|---|---|---|---|---|
| Notched Izod 23°C DAM | 21.8 | 85.2 | 57.5 | 89 | 82.3 |
| Notched Izod -30°C DAM | 12.2 | 24.6 | 16.4 | 75 | 67.5 |
| Notched Izod , -40°C DAM | 12.3 | 17.6 | 14.4 | 36.6 | 31.7 |

The results in Table 4 suggest that even when the total elastomer level is high, a substantial improvement in ductility is achieved at -30°C only at a high neutralization level (Examples 1A and 3B).

### EXAMPLE 5

A composition was made according to the process of Example 1A except that the composition of the elastomer phase was varied. A different maleic anhydride-grafted EP was used so as to keep the level of maleic anhydride contributed by the maleic anhydride-grafted EP the same as in Example 1A while varying the level of total elastomer. In Example 1A, the maleic anhydride-grafted EP was 0.9% MAh and with an MFI of 20 @ 280°C, 2.16Kg. In Example 5, the maleic anhydride-grafted EP was 2% MAh with an MFI of 1 @ 190°C, 2.16Kg. The percent of neutralization was maintained at 150% in Example 5.

Molded flex bars comprised of the composition of Example 5 were tested for impact resistance according to the Notched Izod test method used in Example 1. The test flex bars (5 to 10 bars per test) were molded according to ISO 294 in a standard injection molding machine with a melt temperature of about 285°C, a mold temperature of about 75°C, and a hold pressure was 85 MPa. The bars were tested in the dry-as-molded state at 23°C, -30°C and -40°C. The average impact energy measurements as per ISO 180 are reported in units of KJ/m² in Table 5 and are compared against the results of Example 1A.

**Table 5.**

| Example | 1A | 5 |
|---|---|---|
| Composition (weight %) | | |
| Nylon 6 | 18.2 | 18.2 |
| Ionomer | 7.8 | 7.8 |
| Nylon 6,6 | 55.0 | 64.0 |
| g-Mah-EP | 19.0 | 10.0 |
| Total elastomer % | 26.8 | 17.8 |

| Properties | | |
|---|---|---|
| Notched Izod 23°C DAM | 89.0 | 88.4 |
| Notched Izod -30°C DAM | 75.0 | 30.7 |
| Notched Izod , -40°C DAM | 36.6 | 24.6 |

The results in Table 5 suggest that at the same level of neutralization, and even at the same level of maleic anhydride contributed by the maleic anhydride-grafted EP, the total level of elastomer contributes to the substantial increase in ductility at -30°C.

### EXAMPLE 6

Compositions were made according to the process of Example 1 except that the nature of the nylon feedstock was varied. In Example 6A, the ionomer of Example 1, the EP grafted with maleic anhydride, and nylon 6,6 (VN 138 to 149 in formic acid, according to ISO 307) were melt blended according to the process used in Example 1B. In Example 6B, the ionomer of Example 1, EP grafted with maleic anhydride, and nylon 6 (VN 131- 153 in sulfuric acid, according to DIN 53727) were melt blended according to the process used in Example 1B. These compositions were compared against the composition of Example 1A in which the ionomer, nylon 6 (VN 131-153 in sulfuric acid, according to DIN 53727), EP grafted with maleic anhydride, and nylon 6,6 (VN 138 to 149 in formic acid, according to ISO 307) were blended. In Examples 6A, 6B and 1A, the EP was grafted with 0.9% maleic anhydride and it had an MFI of 20 @ 280°C, 2.16Kg.

Molded flex bars comprised of the compositions 6A and 6B were tested for impact resistance according to the Notched Izod test method used in Example 1. The test flex bars (5 to 10 bars per test) were molded according to ISO 294 in a standard injection molding machine with a melt temperature of about 285°C, a mold temperature of about 75°C, and a hold pressure was 85 MPa. The bars were tested in the dry-as-molded state at 23°C, -30°C and -40°C. In Table 6, the average impact energy measurements as per ISO 180 are reported in units of KJ/m² for Examples 6A and 6B and for Example 1A.

**Table 6.**

| Example | 6A | 6B | 1A |
|---|---|---|---|
| Composition (weight %) | | | |
| Nylon 6 | 0 | 73.2 | 18.2 |
| Ionomer | 7.8 | 7.8 | 7.8 |
| Nylon 6,6 | 73.2 | 0 | 55.0 |
| g-MAh-EP | 19.0 | 19.0 - | 19.0 |

| Properties | | | |
|---|---|---|---|
| Notched Izod 23°C DAM | 85.1 | 20 | 89 |
| Notched Izod -30°C DAM | 29.8 | 19 | 75 |
| Notched Izod , -40°C DAM | 23.7 | 17 | 36.6 |

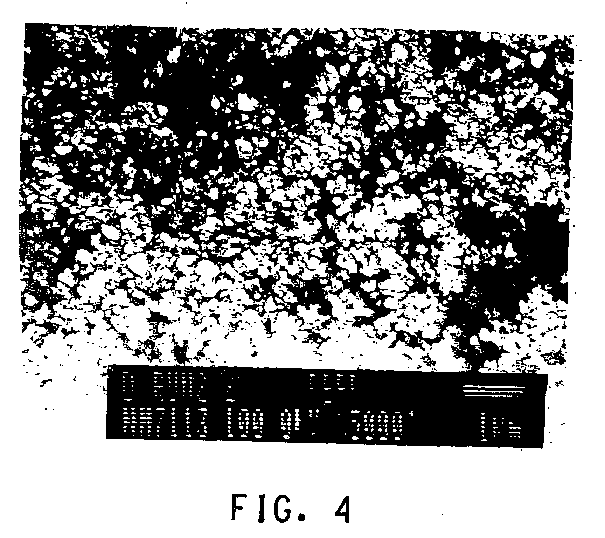

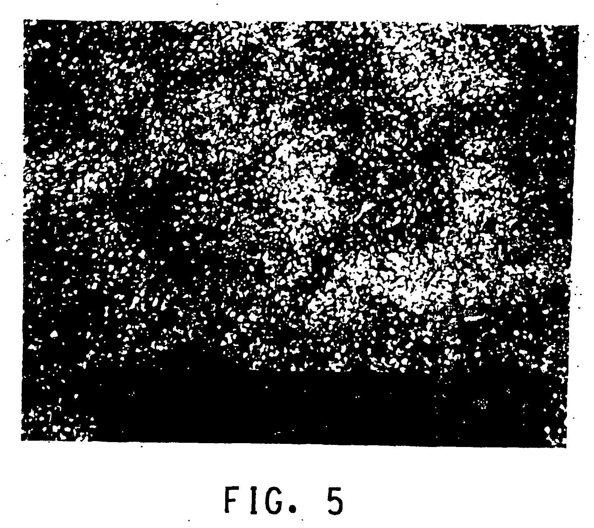

The results in Table 6 show that a substantial increase in ductility, measured according to the Notched Izod, is achieved at -30°C only when the feedstock is a mixture of nylon 6 and nylon 6,6. A TEM image of the composition of Example 6B, as shown in Figure 5, where only nylon 6 was included in the composition, shows a quasi-monodisperse morphology (very similar to that of the blend shown in Figure 1) with a particle size range between 0.02 and 0.2 µm. A TEM image of the composition of Example 6A (Figure 4), where only nylon 6,6 was included in the composition, shows a morphology which looks like interpenetrating networks similar to the composition shown in Figure 2, with average particle size between 0.02 and 0.3 µm. However, it was only when both nylon 6 and nylon 6,6 were present in the composition that a morphology was obtained that looks like interpenetrating networks and that the substantial improvement in ductility at -30°C was achieved.

## Claims

1. A multiphase polymer composition having increased impact resistance at low temperatures, said composition comprising a blend of
(1) an ionomer at least partially dispersed in nylon 6, said ionomer being formed from a partially neutralized acid ethylene acid interpolymer precursor, the acid copolymer precursor having polymerized in-chain units derived from the monomers comprising:
(a) ethylene,
(b) 2 to 25 weight percent of an acid selected from the group of acrylic acid, methacrylic acid, and mixtures thereof,
(c) 0.1 to 15 weight percent of a dicarboxylic acid monomer selected from the group consisting of maleic acid, fumaric acid, itaconic acid, maleic anhydride, itaconic anhydride, a C₁-C₄-alkyl half ester of maleic acid, and a mixture of these dicarboxylic acid monomers,
(d) 0-40 weight percent of a C1-C8-alkyl alkyl acrylate,
the ionomer being formed by neutralization of from about 5 to 90 percent of the total number of carboxylic acid units in the copolymer with metal ions,
with the proviso that the total of acrylic acid, methacrylic acid and dicarboxylic acid monomer is from 4 to 26 weight percent of the acid copolymer precursor, and with the further proviso that the total comonomer content not exceed 50 weight percent of the acid copolymer precursor,
the ionomer having a melt index of from 0.01 to 100 grams/10 minutes;
(2) a polyamide other than nylon 6
(3) composition that includes an elastomer selected from the group of EP, EPDM and styrenic thermoplastic elastomer, which elastomer is grafted with 0.05 to 3 weight percent of a carboxylic acid or any anhydride thereof;
wherein the ratio of the number of moles of metal ions in the ionomer to the number of moles of the carboxylic acid in the grafted EP, EPDM or styrenic thermoplastic elastomer is at least 1.0.

2. The composition of claim 1 where component (1) can be made by melt- or cube-blend of the ionomer and nylon 6.

3. The composition of claim 1, wherein the elastomer portion represents more than 20% based on the total weight of the formulation.

4. The composition of claim 1, wherein the ionomer is formed by neutralization of from about 10 to 70 percent of the total number of carboxylic acid units in the copolymer with a metal ion.

5. The composition of claim 1 wherein the metal ion is selected from the group of zinc, magnesium, manganese and mixtures thereof, alone or in combination with other metal cations such as, but not limited to sodium, potassium or lithium.

6. The composition of claim 1, wherein the polyamides in component (2) are aliphatic or partially aliphatic nylons, and is preferably nylon 6,6.

7. The composition of claim 1, wherein component (3) contains an EP or EPDM grafted with a carboxylic acid or an anhydride precursor selected from the group consisting of maleic acid, fumaric acid, itaconic acid, maleic anhydride, itaconic anhydride, a C₁-C₄-alkyl half ester of maleic acid, and mixtures thereof, and is preferably EP grafted with maleic anhydride.

8. The composition of claim 1, wherein the ratio of the number of moles of metal ions in the ionomer to the number of moles of the carboxylic acid or any anhydride precursor in the grafted EP or EPDM is at least 1.0, and preferably at least 1.5.

9. A molded article made from any of the compositions of claims 1 to 8.

## Patentansprüche

1. Multiphasenpolymerzusammensetzung mit vergrößerter Schlagzähigkeit bei niedrigen Temperaturen, wobei die Zusammensetzung ein Gemisch von
(1) einem Ionomer, zumindest teilweise in Nylon 6 dispergiert, wobei das Ionomer aus einem Ethylen-Säure-Interpolymer-Vorprodukt mit teilweise neutralisierter Säure erzeugt ist, wobei das Säurecopolymervorprodukt polymerisierte Einheiten in der Kette, abgeleitet von den Monomeren, umfassend:
(a) Ethylen,
(b) 2 bis 25 Gewichtsprozent einer Säure, ausgewählt aus der Gruppe von Acrylsäure, Methacrylsäure und Gemischen davon,
(c) 0,1 bis 15 Gewichtsprozent eines Dicarbonsäuremonomers, ausgewählt aus der Gruppe, bestehend aus Maleinsäure, Fumarsäure, Itaconsäure, Maleinsäureanhydrid, Itaconsäureanhydrid, einem C₁-C₄-Alkylhalbester von Maleinsäure und einem Gemisch dieser Dicarbonsäuremonomere,
(d) 0-40 Gewichtsprozent eines C₁-C₈-Alkylalkylacrylats,
aufweist, wobei das Ionomer durch Neutralisation von etwa 5 bis 90 Prozent der Gesamtzahl der Carbonsäureeinheiten in dem Copolymer mit Metallionen erzeugt ist,
mit der Maßgabe, daß die Gesamtmenge von Acrylsäure, Methacrylsäure und Dicarbonsäuremonomer von 4 bis 26 Gewichtsprozent des Säurecopolymervorprodukts beträgt, und mit der weiteren Maßgabe, daß der Gesamtcomonomergehalt 50 Gewichtsprozent des Säurecopolymervorprodukts nicht überschreitet,
wobei das Ionomer einen Schmelzindex von 0,01 bis 100 Gramm/10 Minuten hat;
(2) einem Polyamid anders als Nylon 6;
(3) einer Zusammensetzung, die ein Elastomer, ausgewählt aus der Gruppe von EP, EPDM und thermoplastischem Styrolelastomer, einschließt, welches Elastomer mit 0,05 bis 3 Gewichtsprozent einer Carbonsäure oder eines Anhydrids davon gepfropft ist;
umfasst, wobei das Verhältnis der Anzahl von Molen von Metallionen in dem Ionomer zu der Anzahl von Molen der Carbonsäure in dem gepfropften EP, EPDM oder thermoplastischem Styrolelastomer mindestens 1,0 beträgt.

2. Zusammensetzung nach Anspruch 1, wo Komponente (1) durch Schmelz- oder Würfelmischen von dem Ionomer und Nylon 6 hergestellt werden kann.

3. Zusammensetzung nach Anspruch 1, wobei der Elastomeranteil mehr als 20%, bezogen auf das Gesamtgewicht der Formulierung, darstellt.

4. Zusammensetzung nach Anspruch 1, wobei das Ionomer durch Neutralisation von etwa 10 bis 70 Prozent der Gesamtzahl von Carbonsäureeinheiten in dem Copolymer mit einem Metallion erzeugt wird.

5. Zusammensetzung nach Anspruch 1, wobei das Metallion aus der Gruppe von Zink, Magnesium, Mangan und Gemischen davon, allein oder in Kombination mit anderen Metallkationen wie beispielsweise, ohne aber darauf beschränkt zu sein, Natrium, Kalium oder Lithium, ausgewählt ist.

6. Zusammensetzung nach Anspruch 1, wobei die Polyamide in Komponente (2) aliphatische oder teilweise aliphatische Nylons sind und vorzugsweise Nylon 6,6 sind.

7. Zusammensetzung nach Anspruch 1, wobei Komponente (3) ein EP oder EPDM, gepfropft mit einer Carbonsäure oder einem Anhydridvorprodukt, ausgewählt aus der Gruppe, bestehend aus Maleinsäure, Fumarsäure, Itaconsäure, Maleinsäureanhydrid, Itaconsäureanhydrid, einem C₁-C₄-Alkylhalbester von Maleinsäure und Gemischen davon, enthält und vorzugsweise EP, gepfropft mit Maleinsäureanhydrid, ist.

8. Zusammensetzung nach Anspruch 1, wobei das Verhältnis der Anzahl von Molen von Metallionen in dem Ionomer zu der Anzahl von Molen der Carbonsäure oder eines Anhydridvorprodukts in dem gepfropften EP oder EPDM mindestens 1,0 und vorzugsweise mindestens 1,5 beträgt.

9. Formgegenstand, hergestellt aus einer der Zusammensetzungen nach den Ansprüchen 1 bis 8.

## Revendications

1. Composition polymère multiphase ayant une résistance améliorée aux chocs aux basses températures, ladite composition comprenant un mélange de
(1) un ionomère au moins partiellement dispersé dans du nylon 6, l'ionomère étant formé à partir d'un précurseur interpolymère d'acide éthylène acide partiellement neutralisé, le précurseur copolymère d'acide ayant des motifs polymérisés à l'intérieur de la chaîne dérivés des monomères comprenant:
(a) de l'éthylène,
(b) de 2 à 25 pour cent en poids d'un acide choisi parmi le groupe de l'acide acrylique, de l'acide méthacrylique, et de mélanges de ceux-ci,
(c) de 0,1 à 15 pour cent en poids d'un monomère d'acide dicarboxylique choisi parmi le groupe constitué de l'acide maléique, acide fumarique, acide itaconique, anhydride maléique, anhydride itaconique, un demi-ester d'alkyle en C₁₋₄ de l'acide maléique, et d'un mélange de ces monomères d'acide dicarboxylique,
(d) de 0 à 40 pour cent en poids d'un acrylate d'alkyl alkyle en C₁₋₈,
l'ionomère étant formé par la neutralisation d'environ 5 à 90 pour cent du nombre total des motifs d'acide carboxylique dans le copolymère avec des ions métalliques,
à condition que le total de l'acide acrylique, de l'acide méthacrylique, et du monomère d'acide dicarboxylique soit de 4 à 26 pour cent en poids du précurseur copolymère d'acide, et à condition aussi que la teneure totale en comonomère ne dépasse pas 50 pour cent en poids dudit précurseur copolymère d'acide,
l'ionomère ayant un indice de fluidité de 0,01 à 100 grammes/10 minutes;
(2) un polyamide autre que le nylon 6, et
(3) une composition qui inclut un élastomère choisi parmi le groupe de l'EP, EPDM et d'un élastomère thermoplastique styrénique, lequel élastomère est greffé avec 0,05 à 3 pour cent en poids d'un acide carboxylique ou de n'importe quel anhydride de celui-ci;
dans lequel le rapport du nombre de moles des ions métalliques dans l'ionomère au nombre de moles de l'acide carboxylique dans l'EP, l'EPDM ou l'élastomère thermoplastique styrénique greffé est au moins de 1,0.

2. Composition selon la revendication 1, où le composant (1) peut être fabriqué par mélange à l'état fondu ou en cube de l'ionomère et du nylon 6.

3. Composition selon la revendication 1, dans laquelle la portion d'élastomère représente plus de 20% basés sur le poids total de la formulation.

4. Composition selon la revendication 1, dans laquelle l'ionomère est formé par la neutralisation d'environ 10 à 70 pour cent du nombre total de motifs d'acide carboxylique dans le copolymère avec un ion métallique.

5. Composition selon la revendication 1, dans laquelle l'ion métallique est choisi parmi le groupe du zinc, magnésium, manganèse et des mélanges de celui-ci, seul ou en association avec d'autres cations métalliques tels que, mais sans être limités au sodium, au potassium ou au lithium.

6. Composition selon la revendication 1, dans laquelle les polyamides dans le composant (2) sont des nylons aliphatiques ou partiellement aliphatiques, et sont de préférence du nylon 6,6.

7. Composition selon la revendication 1, dans laquelle le composant (3) contient un EP ou EPDM greffé avec un acide carboxylique ou un précurseur d'anhydride choisi parmi le groupe constitué de l'acide maléique, acide fumarique, acide itaconique, anhydride maléique, anhydride itaconique, d'un demi-ester d'alkyle en C₁₋₄ d'acide maléique, et de mélanges de ceux-ci, et est de préférence de l'EP greffé avec de l'anhydride maléique.

8. Composition selon la revendication 1, dans laquelle le rapport du nombre de moles des ions métalliques dans l'ionomère au nombre de moles de l'acide carboxylique ou de n'importe quel précurseur d'anhydride dans l'EP ou l'EPDM greffé est d'au moins 1,0, et de préférence d'au moins 1,5.

9. Article moulé fabriqué selon l'une quelconque des compositions des revendications 1 à 8.
